# EUROPÄISCHE PATENTSCHRIFT

(11) **EP 2 314 345 B1**
(45) Veröffentlichungstag und Bekanntmachung des Hinweises auf die Patenterteilung: **15.01.2014**
(21) Anmeldenummer: 10010194.8
(22) Anmeldetag: 22.09.2010
(51) Int. Cl.: A61N 1/04, A61B 5/0408, A61B 5/04, A61B 5/0478, A61B 5/0492, A61B 5/0496, A61B 5/0448

(54) **Medizinische, selbstklebende, flexible Einmalelektrode und Verfahren zu deren Herstellung**
Medicinal, self-adhesive, flexible disposable electrode and method for producing same
Électrode médicale à usage unique, auto-adhésive et flexible et son procédé de fabrication

(30) Priorität: 02.10.2009 DE 102009048041
(43) Veröffentlichungstag der Anmeldung: 27.04.2011
(73) Patentinhaber: Covidien AG, 8212 Neuhausen am Rheinfall (CH)
(72) Erfinder: Anz, Johannes, 38828 Wegeleben/Adersleben (DE)
(74) Vertreter: Olsson, Carl H.S.

(56) Entgegenhaltungen:
- EP-A1- 2 033 575
- DE-U1- 29 819 246
- US-A- 5 265 579
- US-B1- 6 366 795

## Beschreibung

Die Erfindung bezieht sich auf eine medizinische, selbstklebende, flexible Einmalelektrode für die nicht-invasive Zu- oder Ableitung elektrischer Signale über die Haut eines Menschen oder Tieres. Derartige Elektroden kommen in der Praxis als sogenannte Haut-Elek-troden zur Anwendung. Deren Aufgabe ist es, für die unterschiedlichsten klinischen Anwendungen, wie Elektrokardiographie (EKG), Elektroenzephalographie (EEG), Elektro-myogramm (EMG), Oberflächenelektrodenmyographie (OEMG), Elektrookulographie (EOG), Impedanzkardiographie (IKG), Muskelstimulation, Elektro-Impedanz-Tomographie (EIT) usw., über die unverletzte Haut elektrische Signale ab- oder einzuleiten. Die Elektroden sind nur zu einem einmaligen Gebrauch bestimmt. Außer der geforderten guten Signalübertragung spielt bei diesen Elektroden der Kostenfaktor eine nicht unwesentliche Rolle.

Bekannt ist der Einsatz von Einmalelektroden auf Basis von Hydrogelen, als Solid- oder Liquidgelektroden.

Bei den sogenannten Solidgelelektroden besteht der Hydrogelkörper aus einem Polymernetzwerk, in welchen die ionisch leitfähige, wässrige Lösung eingebettet ist. Solidgele kommen grundsätzlich ohne eine mechanische Verankerung durch ein vorgegebenes Flächengebilde aus. Bekannt ist hingegen, dass die Affinität von Hydrogelen zu üblichen Oberflächen, wie Kunststoffoberflächen oder einigen Permanentklebstoffen, so begrenzt ist, dass sich die Gele während der Applikation (z.B. durch Quellung infolge Schweißabsorption) leicht vom Elektrodenkörper lösen und beim Entfernen der Elektrode auf der Haut verbleiben können. Aus der DE 199 18 963 A1 ist eine Solidgelelektrode bekannt, die mit einer vliesartigen planen Unterlage ausgerüstet ist, durch die die Gelkörperhaftung auf dem Untergrund der Elektrodenkonstruktion verstärkt werden soll.

Bei der Familie der Liquidgelelektroden stellt ein saugfähiger Aufnahmekörper (Gelkissen), der mit einer ionisch leitfähigen, mindestens gegen ihr Ausfließen unter normaler Schwerkrafteinwirkung angedickten Flüssigkeit (dem Liquidgel) getränkt ist, den direkten Hautkontakt her, während die weitere Signalübertragung wie bei der Solidgelelektrode dann mittels eines das Gel kontaktierenden, elektrisch leitenden Sensors erfolgt, welcher dann seinerseits mittels metallisch leitender Verbindungen mit dem entsprechenden elektronischen Gerät als dem eigentlichem Signallieferanten und/oder -empfänger verbunden ist.

In der US 6 366 795 B1 sind biomedizinische Einmalelektroden offenbart, die mit einer Elektrolytformulierung mit verbesserter Hautdurchdringung und Leitfähigkeit ausgerüstet sind. Die Elektroden besitzen ein aus Kunststoff bestehendes Gehäuse mit einem elektrischen Anschluss. Eine Kammer innerhalb des Gehäuses ist mit Elektrolyt als Gel gefüllt.

Die untere, hautseitige Öffnung des Gehäuses ist mittels einer porösen Membran verbunden, die mit einem abziehbaren Schutzfilm abgedeckt ist.

Das Gehäuse besitzt eine ringförmige Transformationszone zur Veränderung der Form des Gehäuses. Durch einen Fingerdruck wird das domartige Dach des Gehäuses nach unten in eine stabile Position bewegt, wodurch Druck auf das Gel ausgeübt, so dass dieses durch die Poren auf die Hautkontaktstelle gelangt.

Bekannt ist ein Verfahren zur Herstellung von biomedizinischen Mehrfachelektroden zur Einmalverwendung (EP 2 033 575 A1). Die Elektrodenkörper der hergestellten Mehrfachelektroden sind mit einem Sensor mit einem externen, metallisch leitenden Anschlussglied mechanisch und elektrisch leitend verbunden, wobei die in Richtung Haut zeigende Kontaktfläche des Sensors mit einer dünnen Silber/Silberchlorid-Schicht überzogen ist.

Der Gelträger für das Hydrogel besteht aus flexiblem, nassfestem, saugfähigem und hautverträglichem Textilmaterial. Die Mehrfachelektroden sind mit einem einheitlichen Trägermaterial und einer hautseitigen Abdeckung der Gelkörper ausgerüstet.

Aus der DE 299 15 267 U1 ist eine Elektrode mit einem Gelschwamm zur Aufnahme einer leitfähigen Paste als Liquidgel bekannt.

Das meistverbreitete Material zur Aufnahme eines Liquidgels ist ein retikulierter, dadurch offenzelliger Polyurethanschaum. Derartige Gelkissen mit dauerelastischem Verhalten haben ein gutes Aufnahmevermögen für die Gele. Ihr Nachteil besteht darin, dass ihre offenen, scharfen Zellkanten bei der praktischen Anwendung auf empfindlicher Haut mechanische Reizungen verursachen können, die sich in Irritation der betreffenden Stelle und unangenehmem Juckreiz äußern können.

In der Praxis nimmt die EMG-Messung gegenüber der bislang dominierenden EKG-Messung, die sich lediglich auf das vom Herzmuskel erzeugte Elektrosignal beziehen soll, zunehmend breiteren Raum ein. Dadurch besteht ein erhöhter Bedarf an kleinformatigen Elektroden zur Einmalverwendung. So hat sich beispielsweise der Bedarf an Hautelektroden für Messungen der Augenbewegungen (REM) in den letzten zwanzig Jahren etwa verfünffacht und im Anästhesiebereich in etwa verdreifacht. Die Applikationszeiten liegen in einem Bereich von wenigen Minuten bis zu einigen Tagen. Daher stellt das Problem einer sowohl stabilen Hauthaftung über kurze wie längere Zeiträume wie auch einer schmerzlosen Entfernbarkeit nach der Applikation hohe Anforderungen an den Aufbau der Elektroden. Zu berücksichtigen ist dabei, dass nur eine kleine Klebfläche zur Verfügung steht und eine hohe Signalqualität gefordert wird.

Außerdem müssen die Elektroden nach einer längeren Zwischenlagerung, z.B. von 2 bis 3 Jahren, noch die Forderungen der Güte der Signalübertragung von Gelen (bei möglichst geringem Fremdpotentialeinfluss) gemäß Standard ANSI/AAMI EC-12 erfüllen.

Sensoren auf Basis metallischer Silberfolie haben den Nachteil eines hohen, damit kostenintensiven Silberverbrauchs im Verhältnis zur Oberfläche, die mit dem Hydrogel in leitender Verbindung steht (ca. 2,5 g Silber je mm² wirksamer Leitfläche zum Gelkörper) und sind für kleinformatige Elektroden gebräuchlich. Die Hydrogele selbst besitzen gewöhnlich eine gute elektrische Leitfähigkeit und erzeugen je nach Art verschieden niedrige Hautimpedanzen.

Der Erfindung liegt die Aufgabe zugrunde, eine kleinformatige Einmalelektrode zu schaffen, die die Nachteile bekannter Elektroden vermeidet und sich durch einen einfachen Aufbau, eine kostengünstige Herstellung und gute anwendungstechnische Eigenschaften auszeichnet.

Erfindungsgemäß wird die Aufgabe durch die im Anspruch 1 angegebenen Merkmale gelöst. Vorteilhafte Ausgestaltungen der Elektrode sind Gegenstand der Ansprüche 2 bis 10.

Im Anspruch 11 ist ein Verfahren zur Herstellung dieser Elektroden angegeben. Die Ansprüche 12 bis 14 beziehen sich auf vorteilhafte Weiterbildungen der Verfahrensweise. Die vorgeschlagene Elektrode besteht aus einem selbstklebend ausgerüsteten Trägermaterial mit einer Fläche von 100 bis 850 mm², einem mit dem Trägermaterial fest verbundenen elektrisch leitenden Sensor der mit einem externen, metallisch leitenden Anschlussglied mechanisch und elektrisch leitend verbunden ist, deren freiliegende Anschlussstellen bei Bedarf nach außen hin elektrisch isolierend abgedeckt sind. Die in Richtung Haut zeigende Kontaktfläche des Sensors ist mit einer dünnen Silber-Silber-chlorid -Schicht überzogen. Zur Elektrode gehört außerdem noch ein Gelträger aus flexiblem, nassfestem, saugfähigem und hautverträglichem Textilmaterial, dessen in Richtung Sensor zeigende Kontaktfläche mindestens die gleiche Fläche wie die in Richtung Haut zeigende Kontaktfläche des Sensors aufweist. Vorzugsweise kann der Sensor auch aus Kunststoff bestehen. Dieser kann per se leitfähig sein, also elektrisch leitende Zusatzstoffe enthalten, oder aus einem nicht elektrisch leitfähigen Kunststoffkörper gebildet sein, wobei die Leitfähigkeit zwischen hautseitiger Ableitfläche und externer Ableitung über eine auf den Kunststoffkörper aufgebrachte Metallbeschichtung erfolgt. In bestimmten Anwendungsfällen kann es ausreichend sein, wenn nur die in Richtung Haut zeigende Kontaktfläche des Sensors mit einer dünnen Silber/Silberchlorid-Schicht überzogen ist. Technologisch ist dies jedoch mit einem größeren Aufwand verbunden.

Der Silberanteil der Silber-Silberchlorid-Schicht im Verhältnis zur Oberfläche, die mit dem Hydrogel in leitender Verbindung steht, beträgt 0,02 bis 0,15 mg/mm². Das Verhältnis der Größe der Kontaktflächen von Sensor zu Gelträger liegt in einem Bereich von 1:1 bis 1:2,5. Der Gelträger ist mit einem leitfähigen Hydrogel getränkt, derart, dass ein 0,2 bis 3,5 mm dicker Gelkörper gebildet ist. Hautseitig ist die Elektrode mit einer ablösbaren Abdeckung mit einer trogförmigen Ausnehmung zur Aufnahme des überstehenden Gelkörpers ausgerüstet. Die erfindungsgemäße Elektrode besteht ausschließlich aus vorgenannten Bauteilen. Durch die Kombination vorgenannter Merkmale wird eine Elektrode erhalten, die hervorragende anwendungstechnische Eigenschaften besitzt und sich als Massenartikel kostengünstig herstellen lässt. Von besonderem Vorteil ist die geringe Einsatzmenge für die Silber-Silberchlorid-Beschichtung, bezogen auf die die Haut bedeckende Kontaktfläche des Gelträgers.

Die begrenzte Geometrie des gesamten Elektrodenkörpers ermöglicht auch neuartige Anwendungen zur Hautableitung von Muskelsignalen außerhalb der klassischen EKG-Ableitung. Überraschenderweise zeigte sich, dass das niedrige Verhältnis der Fläche des mit Hydrogel versetzten (relativ kleinen) Gelkörpers in Relation zur wirksamen Sensoroberfläche eine gute Signalübertragung ermöglicht. Der Gelträger besteht vorzugsweise aus einem Gebilde aus gewebten oder ungewebten Fasern mit einem Masseanteil an Fasern von 5 bis 25%. Das Aufnahmevermögen für Hydrogel liegt somit zwischen 75 und 95%.

Trotz des vergleichsweise niedrigen Einsatzes an Silber wird eine hohe Güte einer eigenpotentialarmen Signalübertragung über eine stabile, aber extrem dünne Ag/AgCI-Oberfläche erreicht. Die Anforderungen der Norm ANSI/AAMI EC-12 werden somit in ausreichendem Maße erfüllt. Der niedrige Silberverbrauch ist einerseits aus Kostengründen von Vorteil und andererseits auch aus Umweltschutzgründen, da die Elektroden nach einmaligem Gebrauch in aller Regel entsorgt werden müssen.

Trotz der kleinformatigen Ausführung der Elektroden ist die zur Verfügung stehende Applikationsfläche ausreichend, um eine stabile Haftung der Elektroden auf der Haut sowohl bei kurzen (einige Minuten) als auch bei langen Anwendungen (bis zu mehreren Tagen) zu gewährleisten. Dadurch wird eine stabile Ableitungsqualität sichergestellt. Nach Beendigung der Messungen lassen sich die Elektroden wieder nahezu rückstandsfrei und schmerzlos entfernen. Die Elektroden zeichnen sich durch einen hohen Tragekomfort für den Patienten aus (kein Jucken, keine Hautirritationen), und sind trotz ihrer kleinen Baugröße eine in sich mechanisch stabile Konstruktion, die während der Tragezeiten resistent gegen Einflüsse durch Bewegung des Patienten und Schweißabsorption im Hydrogelkörper ist.

Die vorgeschlagene Einmalelektrode kann entsprechend dem vorgesehenen Anwendungszweck hinsichtlich des Trägermaterials (Schaum, Vlies), der Art des Hydrogels (Solid- oder Liquidgel), unterschiedlich ausgeführt werden. Die Herstellung der Elektroden kann automatisiert innerhalb einer Linie erfolgen.

Der Gelträger kann aus einem Gebilde aus Vliesmaterialien auf Basis synthetischer oder natürlicher Fasern oder Gemischen aus diesen bestehen.

Der Gelträger besitzt beispielsweise ein Flächengewicht von 25 bis 300 g/m², vorzugsweise 35 bis 220 g/m².

Die den Gelträger umgebende Klebfläche zur Fixierung der Elektrode auf der Haut sollte 49 bis 98% der Gesamtfläche des Trägermaterials der Elektrode betragen.

Vorzugsweise wird die Verbindung zwischen Sensor und Gelträger mittels einer thermische Fixierung oder über eine Klebverbindung hergestellt. Dabei sollte die Verbindungsfläche nur bis zu 25% der zur Haut zeigenden Kontaktfläche des Sensors betragen, um nicht die Signalqualität zu beeinträchtigen.

In an sich bekannter Weise besteht das einseitig permanent haftend ausgerüstete Trägermaterial aus einem flexiblen Material auf Basis eines Polymerschaums, einer kompakten Polymerfolie, eines Vlieses oder Gewebes oder aus Kombinationen dieser Materialien.

Der Sensor kann üblicherweise als zweiteilige Druckknopfkombination oder als einteiliger Sensor in Kombination mit einer Litze ausgeführt werden.

Der Teil des Trägermaterials, der die Grifflasche bilden soll, ist an seinen Außenseiten, die die Grifffläche bilden, klebstofffrei. Die mit Klebstoff beschichtete Fläche wird entweder abgedeckt oder der Klebstoff wird entfernt.

In ihrer geometrischen Form wird die Elektrode vor allem in kreisrunder oder ovaler Ausführung hergestellt.

Zur Herstellung der erfindungsgemäßen Elektroden sind folgende Verfahrensschritte vorgesehen, insbesondere in einem sogenannten one-piece-flow-Verfahren.

In eine kontinuierlich zugeführte, mit hautverträglichem Permanentklebstoff beschichtete Bahn aus Trägermaterial mit Originalliner werden üblicherweise innerhalb einer Fertigungslinie an vorbestimmten Stellen Durchbrüche gestanzt, die zur späteren Einbringung oder Befestigung der Sensoren bestimmt sind. Vorzugsweise wird der zum Trägermaterial zugehörige Originalliner nahe einer Kante so eingeschnitten, dass nach Fertigstellung der Elektrode ein auf dem Schaum verbleibender Teil die Grifflasche hautseitig abdeckt. Dabei kann der zur Grifflaschenabdeckung verwendete Linerabschnitt zunächst von der Trägermaterialbahn abgehoben, gewendet und dann mit seiner nicht abweisend ausgerüsteten Seite wieder aufgebracht werden. Der Sensor wird anschließend so in Position gebracht, dass dessen Anschlussstück durch den Durchbruch ragt und am Trägermaterial fixiert werden kann. An das Anschlussstück des Sensors wird ein Gegenstück zur Signalableitung, wie ein Druckknopf oder eine Litze angeschlagen. Danach wird ein vorgefertigter Gelträger von der in Richtung Haut zeigenden Seite her zentrisch über den Sensor aufgedrückt und fixiert.

In den Gelträger wird zur Ausbildung des Gelkörpers eine definierte Menge Hydrogel injiziert.

Handelt es sich bei dem Hydrogel um ein UV-initüerbares, polymerisationsfähiges Gel, so durchläuft die Bahn zusätzlich eine UV-Belichtungsstrecke, um die Polymerisation des Hydrogels zu starten. Nachfolgend werden alle in Richtung Haut zeigenden Flächen der Elektrode mittels eines vorgefertigten lösbaren Liners abgedeckt, wobei in den Liner in der Regel trogförmige Ausnehmungen eingeformt sind, die die Gelkörper umschließen. Als Alternative kann in das Trägermaterial eine Vertiefung, beispielsweise durch Tiefziehen, eingebracht werden, die die über die Planlage der klebenden Trägermaterialseite hinausragende, in Richtung haut zeigenden Teile aufnimmt. Der Liner könnte dann plan bleiben.

Abschließend werden die fertigen Elektroden ausgestanzt.

Die Erfindung soll nachstehend an einem Ausführungsbeispiel näher erläutert werden. In der zugehörigen Zeichnung zeigen:
- Fig.1 1: die erfindungsgemäße Elektrode in kreisrunder Ausführung mit Sensor in Druckknopfkombination ohne hautseitige Abdeckung, als Draufsicht,
- Fig. 2: die Elektrode gemäß Fig.1 mit hautseitiger Abdeckung als Explosionsdarstellung und
- Fig. 3: eine weitere Variante der Elektrode in ovaler Ausführung mit Sensor in Litzenkombination ohne hautseitige Abdeckung, als Draufsicht.

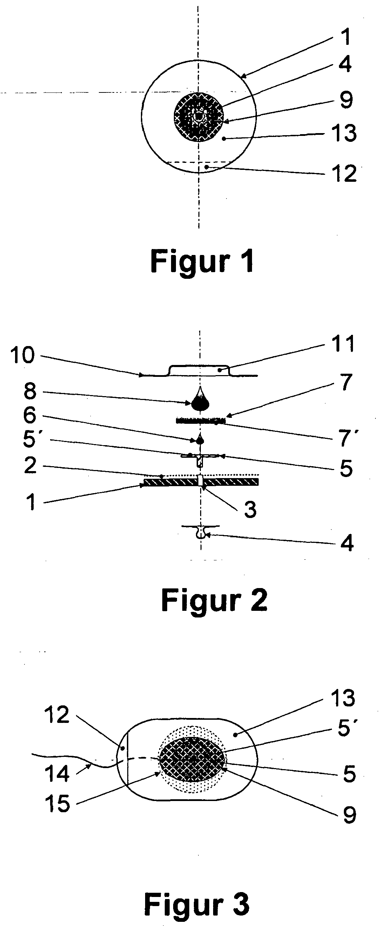

Der in den Figuren 1 und 2 gezeigte Aufbau der erfindungsgemäßen Einmalelektrode kreisrunder Ausführung wird nachfolgend im Zusammenhang mit deren Herstellung erläutert.

Über die Eingabefelder eines computergesteuerten Fertigungsautomaten wird der Fertigungscode der herzustellenden Elektrode eingegeben. Über die installierte Software werden dann auf elektronischem Weg die abgespeicherten Fertigungsdaten ermittelt und die Steuerung des Automaten entsprechend ausgelöst.

In den nicht näher gezeigten Fertigungsautomaten läuft eine mit hautverträglichem Permanentklebstoff 2 beschichtete Polyethylen-Schaumbahn 1 als Endlosbahn mit Liner in einer vorgegebenen Breite ein. Der zum Trägermaterial 1 zugehörige Originalliner wird nahe einer Kante so eingeschnitten, dass ein später auf dem Trägermaterial 1 verbleibender Teil die Grifflasche 12 hautseitig abdeckt, wobei bevorzugt der zur Grifflaschenabdeckung verwendete Linerabschnitt zunächst von der Trägermaterialbahn 1 abgehoben, gewendet und dann mit seiner nicht abweisend ausgerüsteten Seite wieder aufgebracht wird, um einen sicheren Halt während der Applikation zu gewährleisten. Da die Elektrode mit einem zentrisch angeordneten Druckknopf-Anschluss ausgerüstet werden soll, wird in die PE-Schaumbahn mittig ein Durchbruch 3 ausgestanzt (Öffnung: 3 mm Durchmesser). Anschließend werden mindestens die später auf der Haut klebenden Partien der Trägermaterialbahn 1 vom Liner freigelegt.

Zur elektrischen Signalübertragung werden gel- bzw. hautseitig ein mit einer Silber-Silberchlorid-Schicht überzogener Kunststoffsensor 5, dessen in Richtung Haut zeigende Kontaktfläche 5' einen Durchmesser von 10 mm aufweist und an der Gegenseite ein Metalldruckknopf 4 kraftschlüssig befestigt, wobei das Trägermaterial 1 zwischen diesen eingeklemmt wird. Die Sensoroberfläche beträgt 80 mm² und Schichtdicke der Ag/AgCl-Schicht ca. 2 µm. Der Einsatz an reinem Silber liegt bei ca. 3,2 mg, das entspricht 0,04 mg/mm² wirksamer Sensoroberfläche. Im Vergleich dazu liegt der Silbereinsatz bei marktgängigen Schlaflaborelektroden bei ca. 2,5 mg/mm².

Nachfolgend wird der Gelträger 7 auf der Kontaktfläche 5' des Sensors 5 fixiert.

Der Außendurchmesser des Gelträgers 7, der aus einem schwach verdichteten Vlies aus PP-Viskose-Fasern besteht, beträgt 12 mm, ist also geringfügig größer als- der Außendurchmesser der Kontaktfläche 5'. Die eingesetzte Vliesqualität liegt bei 80 g/m². Die Höhe des Gelträgers 7 beträgt 2,8 mm.

Der Gelträger 7 wird mittels eines Schmelzklebstoffes 6 auf der Kontaktfläche 5' des Sensors 5 fixiert. Die Klebstoffmenge 6 wird dosiert mittig auf die Kontaktfläche 5' des Sensors 5 in einer solchen Menge aufgetragen, dass der Klebstoff nicht mehr als 10% der hautseitigen Sensorfläche 5' bedeckt. Der Gelträger 7 wird von oben her zentrisch mit seiner Kontaktfläche 7' auf die Sensorfläche 5' aufgedrückt und fixiert.

Die effektive Klebstofffläche ist insofern von Bedeutung, da ein größerer Flächenauftrag an Klebstoff zu einer Beeinträchtigung der Signalübertragung der Elektrode führen kann.

Das Verhältnis der Größe der Kontaktfläche 5' von Sensor 5 zur Kontaktfläche 7' des Gelträgers 7 beträgt in diesem Fall 1:1,4. Dadurch wird später eine sehr gute direkte Signaldurchleitung zum Sensor 5 gewährleistet.

Nachfolgend wird zur Ausbildung des Gelkörpers 9 eine definierte Menge eines ionisch leitfähigen, rheologisch passend eingestellten Liquidgels 8 in den Gelträger 7 injiziert. Der gebildete Gelkörper 9 besteht aus dem Gelträger 7 und dem injizierten Hydrogel 8. Die injizierte Menge ist so dosiert, dass diese, bezogen auf den Masseanteil an Fasern des Gelträgers, 91 % beträgt. Durch die Faserstruktur des Gelträgers 7 wird das Liquidgel 8 in diesem fixiert.

Zur Abdeckung aller hautseitigen Flächen wird ein ablösbarer Liner 10 aus einer tiefziehfähigen Kunststofffolie zugeführt. In diesen wurde bereits eine trogförmige Ausnehmung 11 (Innendurchmesser: 14 mm und Innenhöhe: 3 mm) eingeformt, die geringfügig größer ist als der Gelkörper 9. Der Liner 10 wird so platziert, dass die trogförmige Ausnehmung 11 den Gelkörper 9 umgibt und die ringförmige Klebfläche 13 der Elektrode bedeckt.

Abschließend wird die fertige kreisrunde Elektrode (Durchmesser 30 mm) ausgestanzt und bis zum Gebrauch dampfdicht verpackt.

Unmittelbar vor der Applikation der Elektrode wird der ablösbare Liner 10 wieder entfernt. Die den Gelkörper 9 umgebende wirksame Klebfläche 13 zur Fixierung der Elektrode auf der Haut beträgt 84% des Trägermaterials 1 der fertigen Elektrode.

Die kleinformatige Ausführung der Elektrode ermöglicht deren Einsatz für EMG-Messung-en, beispielsweise im Schlaflabor.

Für die Bewertung der Eigenleitfähigkeit der Elektrode wird der Impedanzwert ACZ1 (vor Simulation der Defibrillation, ermittelt bei einer Messfrequenz von 10 Hz) zu ca. 110 Ohm nach dem für Einwegelektroden gültigen Standard ANSI/AAMI EC12-2000 mittels Messung an Elektrodenpaaren ermittelt. Dieser Wert liegt damit weit unterhalb der Vorgabe des Standards mit maximal 2.000 Ohm. Die übrigen Standardwerte (insbesondere zum Potentialeinfluss) werden in hoher Qualität eingehalten.

Der Anschluss an den Monitor erfolgt mittels üblicher Klemmadapter über die frei liegenden Druckknöpfe.

Eine Aussage zum Tragekomfort und zur Signalsicherheit wurde in üblicher Weise durch einen praktischen Tragetest ermittelt. Hierzu wurden 10 Probanden unterschiedlichen Hauttyps (in diesem Beispiel im Oberbauchbereich) Elektroden appliziert, Messungen durchgeführt und nach einer Applikationsdauer von 12 Stunden das Trageverhalten beurteilt.

Bei allen Probanden wurde eine ausreichende Hauthaftung der Elektroden ohne Teilablösungen oder Signalstörungen während der Tragezeit festgestellt. Bei Abnahme der Elektrode verbleiben Spuren von Liquidgel auf der Haut, die sich mühelos mittels Zellstoff von der Haut abreiben lassen. Hautreizungen sind nicht aufgetreten.

In der Figur 3 ist eine Elektrode in ovaler Ausführung gezeigt.

Außer der geometrischen Form unterscheidet sich diese Elektrode von der in den Figuren 1 und 2 gezeigten Elektrode durch den Einsatz eines Solidgels anstelle eines Liquidgels. Außerdem wurde als Anschlusselement anstelle eines zweiteiligen Druckknopfes ein einteiliger Sensor 5 mit Litze 14 eingesetzt. Dessen kreisrunde Kontaktfläche ist, wie oben beschrieben, in identischer Weise mit einer Ag/AgCl-Schicht überzogen.

Die Herstellung der Elektrode erfolgt ansonsten analog wie vorstehend zu den Figuren 1 und 2 beschrieben.

Nach der Montage eines ovalen Gelträgers 7 auf der kreisrunden Kontaktfläche 5' des Sensor 5 wird in den Gelträger aus Vlies eine unpolymerisierte Gelmischung auf Acrylatbasis injiziert, die sich im gesamten Gelträger ausbreitet.

Nachfolgend durchläuft die endlose Trägerbahn 1 mit den halbfertigen Elektrodenkörpern eine UV-Belichtungsstrecke. Während des Transportes durch die Belichtungsstrecke polymerisiert das flüssige Gel in-situ im Gelkörper 9 aus.

Anschließend werden alle hautseitigen Flächen mit einem ablösbaren Liner 10 aus einer tiefziehfähigen Kunststofffolie abgedeckt, in den durch Tiefziehen die erforderliche trogförmige Ausnehmung 11 zur Aufnahme des ovalen Gelkörpers 9 bereits eingeformt ist. Auf der von der Haut weg zeigenden Seite der Elektrode wird zur Abdeckung bzw. als Schutz des Anschlusses von Litze 14 am Sensor 5 noch ein kreisrundes Label 15 aus selbstklebender Polymerfolie aufgeklebt.

Abschließend werden dann die ovalen Elektroden ausgestanzt und bis zum Gebrauch dampfdicht verpackt.

Die ovale Solidgelelektrode hat eine Fläche von ca.500 mm².

Das Verhältnis der Größe der Kontaktflächen von Sensor zu Gelträger beträgt 1:1,7.
Flächengewicht des Gelträgers: 120 g/m²
Masseanteil des Gelträgers an Fasern: 14%
Masseanteil an Solidgel: 86%
Dicke des Gelträgers bzw. Gelkörpers: 1,8 mm

Die den ovalen Gelkörper 9 umgebende Klebfläche 13 zur Fixierung der Elektrode auf der Haut beträgt 74 % der Gesamtfläche des Trägermaterials der fertigen Elektrode.

Aufgrund der veränderten Gelsorte (Solidgel anstelle von Liquidgel) ist die Impedanz ACZ1 mit 300 Ohm höher als im oben genannten Beispiel. Demzufolge ist die Signalqualität dieser Elektroden wegen der höheren Impedanzwerte nicht für Anwendungen mit erhöhten Ansprüchen, wie Schlaflabordiagnostik, geeignet.

Das Trageverhalten ist analog wie bei den Elektroden gemäß den Figuren 1 und 2.

### Bezugszeichen

- 1: Polyethylen-Schaumbahn
- 2: Permanentklebstoff 2
- 3: Durchbruch/Öffnung
- 4: Metalldruckknopf
- 5: Sensor
- 5': Kontaktfläche von 5
- 6: Klebstoff
- 7: Gelträger
- 7': Kontaktfläche von 7
- 8: Hydrogel/Liquidgel
- 9: Gelkörper (7 + 8)
- 10: Liner
- 11: trogförmige Ausnehmung
- 12: Grifflasche
- 13: wirksame Klebfläche
- 14: Litze
- 15: Label

## Patentansprüche

1. Medizinische, selbstklebende, flexible Einmalelektrode für die nicht-invasive Zu- oder Ableitung elektrischer Signale über die Haut eines Menschen oder Tieres, bestehend ausschließlich aus einem selbstklebend ausgerüsteten Trägermaterial (1) mit einer Fläche von 100 bis 850 mm², einem mit dem Trägermaterial (1) fest verbundenen leitfähigen Sensor (5), der mit einem externen, metallisch leitenden Anschlussglied mechanisch und elektrisch leitend verbunden ist, wobei mindestens die in Richtung Haut zeigende Kontaktfläche (5') des Sensors (5) mit einer dünnen Silber/Silberchlorid-Schicht überzogen ist, mit einem Silberanteil von 0,02 bis 0,15 mg/mm², einem Gelträger (7) aus flexiblem, nassfestem, saugfähigem und hautverträglichem Textilmaterial, dessen in Richtung Sensor (5) zeigende Kontaktfläche (7') mindestens die gleiche Fläche wie die in Richtung Haut zeigende Kontaktfläche (5') des Sensors (5) aufweist, wobei das Verhältnis der Größe der Kontaktfläche (5') von Sensor (5) zur Kontaktfläche (7') von Gelträger (7) bis zu 1:2,5 beträgt, der Gelträger (7) mit einem leitfähigen Hydrogel (8) getränkt ist, derart, dass ein 0,2 bis 3,5 mm dicker Gelkörper (9) gebildet ist, und einer ablösbaren Abdeckung (10) mit einer trogförmigen Ausnehmung (11) zur Aufnahme des überstehenden Gelkörpers (9).

2. Einmalelektrode nach Anspruch 1, **dadurch gekennzeichnet, dass** der Gelträger (7) aus einem Gebilde aus gewebtem oder ungewebtem textilem Material besteht, wobei der Masseanteil an Fasern 5 bis 25% beträgt und das Aufnahmevermögen für das Hydrogel (8) zwischen 75 und 95% liegt.

3. Einmalelektrode nach Anspruch 2, **dadurch gekennzeichnet, dass** das Gebilde aus textilem Material aus Vliesmaterialien auf Basis synthetischer oder natürlicher Fasern oder Gemischen daraus besteht.

4. Einmalelektrode nach einem der Ansprüche 1 bis 3, **dadurch gekennzeichnet, dass** der Gelträger (7) ein Flächengewicht von 25 bis 300 g/m², vorzugsweise 35 bis 220 g/m², aufweist.

5. Einmalelektrode nach einem der Ansprüche 1 bis 4, **dadurch gekennzeichnet, dass** die den Gelkörper (9) umgebende Klebfläche (13) zur Fixierung der Elektrode auf der Haut 49 bis 98% der Gesamtfläche des Trägermaterials (1) der fertigen Elektrode beträgt.

6. Einmalelektrode nach einem der Ansprüche 1 bis 5, **dadurch gekennzeichnet, dass** der Sensor (5) mit dem Gelträger (7) durch eine thermische Fixierung oder über eine Klebverbindung verbunden ist, wobei die Verbindungsfläche bis zu 25% der in Richtung Haut zeigenden Seite der Kontaktfläche (5') des Sensors (5) einnimmt.

7. Einmalelektrode nach einem der Ansprüche 1 bis 6, **dadurch gekennzeichnet, dass** der Gelkörper (9) mit einem Liquid- oder einem Solidgel ausgerüstet ist.

8. Einmalelektrode nach einem der Ansprüche 1 bis 7, **dadurch gekennzeichnet, dass** das Trägermaterial (1) aus einem flexiblen Material auf Basis eines Polymerschaums, einer kompakten Polymerfolie, eines Vlieses oder Gewebes oder aus Kombinationen dieser Materialien besteht.

9. Einmalelektrode nach einem der Ansprüche 1 bis 8, **dadurch gekennzeichnet, dass** der Sensor als zweiteilige Druckknopfkombination (4, 5) oder als einteiliger Sensor (5) in Kombination mit einer Litze (14) ausgeführt ist.

10. Einmalelektrode nach einem der Ansprüche 1 bis 9, **dadurch gekennzeichnet, dass** ein Teil des Trägermaterials (1) als Grifflasche (12) ausgebildet ist, die beidseitig nicht klebend ist.

11. Verfahren zur Herstellung einer Einmalelektrode nach einem der vorhergehenden Ansprüche durch folgende Verfahrensschritte:
a) in eine kontinuierlich zugeführte mit hautverträglichem Permanentklebstoff (2) beschichtete Bahn aus Trägermaterial (1) mit Originalliner werden innerhalb einer Fertigungslinie an vorbestimmten Stellen Durchbrüche (3) gestanzt, die zur späteren Einbringung oder Befestigung der Sensoren (4, 5) bestimmt sind,
b) der Sensor (5) wird so in Position gebracht, dass dessen Anschlussstück durch den Durchbruch (3) ragt und am Trägermaterial (1) fixiert wird,
c) an den Sensor (5) wird ein Gegenstück zur Signalableitung, angeschlagen,
d) ein vorgefertigter Gelträger (7) wird von der in Richtung Haut zeigenden Seite her zentrisch über den Sensor (5) aufgedrückt und fixiert,
e) in den Gelträger (7) wird zur Ausbildung des Gelkörpers (9) eine definierte Menge Hydrogel (8) injiziert,
f) handelt es sich bei dem Hydrogel (8) um ein polymerisationsfähiges Gel, so durchläuft die Bahn zusätzlich eine UV-Belichtungsstrecke, um die Polymerisation des Hydrogels zu initiieren,
g) nachfolgend werden alle in Richtung Haut zeigenden Flächen mittels eines vorgefertigten lösbaren Liners (10) abgedeckt, wobei in den Liner (10) oder in das Trägermaterial trogförmige Ausnehmungen (11) eingeformt sind, die die Gelkörper (9) umschließen, und
h) abschließend wird die fertige Elektrode ausgestanzt werden.

12. Verfahre nach Anspruch 11, **dadurch gekennzeichnet, dass** der Sensor (5) mit dem Gelträger (7) durch eine thermische Fixierung oder über eine Klebverbindung verbunden wird unter Einhaltung einer Verbindungsfläche von bis zu 25%, bezogen auf die hautseitige Kontaktfläche (5') des Sensors (5).

13. Verfahren nach einem der Ansprüche 11 oder 12, **dadurch gekennzeichnet, dass** als Gegenstück zur Signalableitung der Sensor (5) mit einem Druckknopf (4) oder mit einer Litze (14) verbunden wird.

14. Verfahren nach einem der Ansprüche 11 bis 13, **dadurch gekennzeichnet, dass** der zum Trägermaterial (1) zugehörige Originalliner nahe einer Kante so eingeschnitten wird, dass ein auf dem Trägermaterial verbleibender Teil die Grifflasche (12) an der zur Haut zeigenden Seite abdeckt, wobei der zur Grifflaschenabdeckung verwendete Linerabschnitt zunächst von der Trägermaterialbahn (1) abgehoben, gewendet und dann mit seiner nicht abweisend ausgerüsteten Seite wieder aufgebracht wird.

## Claims

1. Medical, self-adhesive, flexible disposable electrode for the non-invasive supply or capture of electrical signals through the skin of a human or animal, which electrode is composed exclusively of a self-adhesive carrier material (1) with a surface area of 100 to 850 mm², a conductive sensor (5) fixedly connected to the carrier material (1) and mechanically and electrically conductively connected to an external, metallically conductive attachment member, wherein at least the contact surface (5') of the sensor (5) facing in the direction of the skin is coated with a thin layer of silver/silver chloride having a silver content of 0.02 to 0.15 mg/mm², a gel carrier (7) made from flexible, moisture-proof, absorbent and skin-compatible textile material, of which the contact surface (7') facing in the direction of the sensor (5) has at least the same surface area as the contact surface (5') of the sensor (5) facing in the direction of the skin, wherein the ratio of the size of the contact surface (5') of sensor (5) to the contact surface (7') of gel carrier (7) is up to 1:2.5, the gel carrier (7) is impregnated with a conductive hydrogel (8) in such a way that a gel body (9) with a thickness of 0.2 to 3.5 mm is formed, and a removable cover (10) with a trough-shaped recess (11) for receiving the protruding gel body (9).

2. Disposable electrode according to Claim 1, **characterized in that** the gel carrier (7) is composed of a structure of woven or unwoven textile material, wherein the proportion by mass of fibres is 5 to 25%, and the absorption capacity for the hydrogel (8) is between 75 and 95%.

3. Disposable electrode according to Claim 2, **characterized in that** the structure of textile material is composed of nonwoven materials based on synthetic or natural fibres or mixtures thereof.

4. Disposable electrode according to one of Claims 1 to 3, **characterized in that** the gel carrier (7) has a basis weight of 25 to 300 g/m², preferably of 35 to 220 g/m².

5. Disposable electrode according to one of Claims 1 to 4, **characterized in that** the adhesive surface (13), which surrounds the gel body (9) and serves to fix the electrode to the skin, accounts for 49 to 98% of the total surface area of the carrier material (1) of the finished electrode.

6. Disposable electrode according to one of Claims 1 to 5, **characterized in that** the sensor (5) is connected to the gel carrier (7) by thermal fixing or by an adhesive connection, wherein the connection surface occupies up to 25% of the side of the contact surface (5') of the sensor (5) facing in the direction of the skin.

7. Disposable electrode according to one of Claims 1 to 6, **characterized in that** the gel body (9) is provided with a liquid gel or a solid gel.

8. Disposable electrode according to one of Claims 1 to 7, **characterized in that** the carrier material (1) is composed of a flexible material based on a polymer foam, a compact polymer film, a nonwoven or woven material, or combinations of these materials.

9. Disposable electrode according to one of Claims 1 to 8, **characterized in that** the sensor is designed as a two-part push-button combination (4, 5) or as a one-part sensor (5) in combination with a stranded wire (14).

10. Disposable electrode according to one of Claims 1 to 9, **characterized in that** a part of the carrier material (1) is designed as a grip tab (12), which is non-adhesive on both sides.

11. Method for producing a disposable electrode according to one of the preceding claims, by the following method steps:
a) within a production line, passages (3) are punched at predetermined locations into a continuously delivered web of carrier material (1), which has an original liner and is coated with skin-compatible permanent adhesive (2), said passages (3) being intended for subsequent introduction or securing of the sensors (4, 5),
b) the sensor (5) is brought into position such that the attachment piece thereof protrudes through the passage (3) and is fixed on the carrier material (1),
c) a mating piece for signal capture is mounted on the sensor (5),
d) a prefabricated gel carrier (7) is pressed on centrally over the sensor (5), from the side facing in the direction of the skin, and is affixed,
e) a defined amount of hydrogel (8) is injected into the gel carrier (7) in order to form the gel body (9),
f) if the hydrogel (8) is a gel that is capable of polymerization, the web additionally passes through a UV lighting section in order to initiate the polymerization of the hydrogel,
g) all the surfaces facing in the direction of the skin are then covered by means of a prefabricated releasable liner (10), wherein trough-shaped recesses (11) are formed in the liner (10) or in the carrier material and enclose the gel bodies (9), and
h) the finished electrode is finally punched out.

12. Method according to Claim 11, **characterized in that** the sensor (5) is connected to the gel carrier (7) by thermal fixing or by an adhesive connection, maintaining a connection surface of up to 25% relative to the skin-side contact surface (5') of the sensor (5).

13. Method according to either of Claims 11 and 12, **characterized in that**, as mating piece for signal capture, the sensor (5) is connected to a push-button (4) or to a stranded wire (14).

14. Method according to one of Claims 11 to 13, **characterized in that** the original liner belonging to the carrier material (1) is incised near one edge such that a part remaining on the carrier material covers the grip tab (12) on the side facing the skin, wherein the liner portion used to cover the grip tab is firstly lifted from the web of carrier material (1), turned round and then reapplied with its non-repellent side.

## Revendications

1. Electrode médicale autocollante flexible à usage unique permettant d'injecter ou de reprendre des signaux électriques de manière non invasive par l'intermédiaire de la peau d'un homme ou d'un animal,
l'électrode étant constituée exclusivement
d'un matériau de support (1) rendu autocollant dont la surface représente de 100 à 850 mm²,
d'un capteur conducteur (5) relié solidairement au matériau de support (1) et raccordé mécaniquement et de manière électriquement conductrice à un élément externe métallique conducteur de raccordement,
au moins la surface de contact (5') du capteur (5) tournée en direction de la peau étant recouverte d'une mince couche d'argent/chlorure d'argent dont la teneur en argent est de 0,02 à 0,15 mg/mm²,
d'un porte-gel (7) en matériau textile flexible, résistant à l'humidité, absorbant et compatible avec la peau dont la surface de contact (7') tournée en direction du capteur (5) présente au moins la même superficie que la surface de contact (5') du capteur (5) tournée en direction de la peau,
le rapport entre la taille de la surface de contact (5') du capteur (5) et la surface de contact (7') du porte-gel (7) pouvant atteindre 1:2,5,
le porte-gel (7) étant imprégné d'un hydrogel conducteur (8) de manière à former un corps de gel (9) d'une épaisseur de 0,2 à 3,5 mm, et
d'un recouvrement libérable (10) doté d'une découpe (11) en forme de cuvette destinée à reprendre le corps de gel (9) en débord.

2. Electrode à usage unique selon la revendication 1, **caractérisée en ce que** le porte-gel (7) est constitué d'un produit en matériau textile tissé ou non tissé dont la teneur massique en fibres est de 5 à 25 % et dont la capacité de reprise de l'hydrogel (8) est comprise entre 75 et 95 %.

3. Electrode à usage unique selon la revendication 2, **caractérisée en ce que** le produit en matériau textile est constitué de matériaux feutrés à base de fibres naturelles ou synthétiques ou de mélanges de ces fibres.

4. Electrode à usage unique selon l'une des revendications 1 à 3, **caractérisée en ce que** le porte-gel (7) présente un poids par unité de surface de 25 à 300 g/m² et de préférence de 35 à 220 g/m².

5. Electrode à usage unique selon l'une des revendications 1 à 4, **caractérisée en ce que** la surface adhésive (13) qui entoure le corps de gel (9) et qui fixe l'électrode sur la peau représente de 49 à 98 % de la superficie totale du matériau de support (1) de l'électrode prête à l'emploi.

6. Electrode à usage unique selon l'une des revendications 1 à 5, **caractérisée en ce que** le capteur (5) est relié au porte-gel (7) par fixation thermique ou par liaison collée, la surface de liaison occupant jusque 25 % du côté de la surface de contact (5') du capteur (5) tourné en direction de la peau.

7. Electrode à usage unique selon l'une des revendications 1 à 6, **caractérisée en ce que** le corps de gel (9) est doté d'un gel liquide ou d'un gel solide.

8. Electrode à usage unique selon l'une des revendications 1 à 7, **caractérisée en ce que** le matériau de support (1) est constitué d'un matériau flexible à base d'une mousse de polymère, d'une feuille polymère compacte, d'un feutre, d'un tissu ou d'une combinaison de ces matériaux.

9. Electrode à usage unique selon l'une des revendications 1 à 8, **caractérisée en ce que** le capteur est configuré comme combinaison (4, 5) de bouton poussoir en deux parties ou comme capteur (5) d'un seul tenant combiné avec une tresse (14).

10. Electrode à usage unique selon l'une des revendications 1 à 9, **caractérisée en ce qu'**une partie du matériau de support (1) est configurée comme patte de saisie (12) dont aucune face n'est adhésive.

11. Procédé de fabrication d'une électrode à usage unique selon l'une des revendications précédentes par les étapes de traitement suivantes :
a) des perforations (3) destinées à apporter ou fixer ultérieurement les capteurs (4, 5) sont estampées dans une ligne de fabrication en des emplacements prédéterminés d'une bande de matériau de support (1) dotée d'une garniture d'origine, apportée en continu et revêtue d'un adhésif permanent (2) compatible avec la peau,
b) le capteur (5) est amené en position de telle sorte que sa pièce de raccordement déborde à travers la perforation (3) et soit fixée sur le matériau de support (1),
c) une pièce complémentaire de prélèvement de signaux est sertie sur le capteur (5),
d) un porte-gel (7) préfabriqué est repoussé et fixé au centre du côté tourné en direction de la peau du capteur (5),
e) une quantité définie d'hydrogel (8) est injectée dans le porte-gel (7) pour former le corps de gel (9),
f) si l'hydrogel (8) est un gel polymérisable, la bande traverse de plus un parcours d'irradiation par UV pour amorcer la polymérisation de l'hydrogel,
g) ensuite, toutes les surfaces tournées en direction de la peau sont recouvertes par une garniture libérable (10) préfabriquée, des découpes (11) en forme de cuvette qui entourent le corps de gel (9) étant formées dans la garniture (10) ou dans le matériau de support et
h) l'électrode terminée est ensuite estampée.

12. Procédé selon la revendication 11, **caractérisé en ce que** le capteur (5) est raccordé au porte-gel (7) par fixation thermique ou par liaison collée en respectant une surface de liaison qui peut atteindre 25 % de la surface de contact (5') du capteur (5) située du côté de la peau.

13. Procédé selon l'une des revendications 11 ou 12, **caractérisé en ce que** pour former la pièce complémentaire de prélèvement des signaux, le capteur (5) est relié à un bouton poussoir (4) ou à une tresse (14).

14. Procédé selon l'une des revendications 11 à 13, **caractérisé en ce que** la garniture d'origine qui fait partie du matériau de support (1) est découpée à proximité d'un bord de telle sorte que la partie qui reste sur le matériau de support recouvre la patte de saisie (12) sur le côté tourné vers la peau, la partie de garniture utilisée pour recouvrir la patte de saisie étant d'abord relevée de la bande (1) de matériau de support, retournée et ensuite réappliquée par son côté non antiadhésif.
